# EUROPEAN PATENT SPECIFICATION

(11) **EP 3 243 500 B1**
(45) Date of publication and mention of the grant of the patent: **12.02.2020**
(21) Application number: 17162627.8
(22) Date of filing: 23.03.2017
(51) Int. Cl.: A61K 6/08, A61K 6/087, A61C 13/00, A61K 6/00, A61C 13/08, A61C 13/09, A61C 13/087

(54) **SOLID FREEFORM FABRICATION MATERIAL SET, METHOD OF MANUFACTURING SOLID FREEFORM FABRICATION OBJECT, AND DEVICE FOR MANUFACTURING SOLID FREEFORM FABRICATION OBJECT**
FESTFREIFORMFERTIGUNGSMATERIALSATZ, VERFAHREN ZUR HERSTELLUNG EINES FESTFREIFORMFERTIGUNGSOBJEKTS UND VERFAHREN ZUR HERSTELLUNG EINES FESTFREIFORMFERTIGUNGSOBJEKTS
ENSEMBLE DE MATÉRIAU DE FABRICATION DE FORME LIBRE SOLIDE, PROCÉDÉ DE FABRICATION D'UN OBJET DE FABRICATION DE FORME LIBRE SOLIDE ET DISPOSITIF DE FABRICATION D'UN OBJET DE FABRICATION DE FORME LIBRE SOLIDE

(30) Priority: 10.05.2016 JP 2016094349
(43) Date of publication of application: 15.11.2017
(73) Proprietor: Ricoh Company, Ltd., Tokyo 143-8555 (JP)
(72) Inventor: SAITO, Takuya, Ohta-ku, Tokyo 143-8555 (JP); WATANABE, Masaki, Ohta-ku, Tokyo 143-8555 (JP); SAKURAI, Yoichi, Ohta-ku, Tokyo 143-8555 (JP); NIIMI, Tatsuya, Ohta-ku, Tokyo 143-8555 (JP)
(74) Representative: White, Duncan Rohan

(56) References cited:
- US-A1- 2004 251 574
- US-A1- 2005 049 739
- US-A1- 2005 079 086
- US-B2- 6 821 462

## Description

### Technical Field

The present invention relates to a solid freeform fabrication material set, a method of manufacturing a solid freeform fabrication object, and a device for manufacturing a solid freeform fabrication object.

### Description of the Related Art

Dental prosthesis (prosthetic tooth) is typically made of metal material such as cobalt-chrome alloy, ceramic material such as zirconia, and organic material such as hybrid resins complexed with fillers.

These prosthetic teeth help to improve occlusion ability in the case of functional failure. However, most of the materials for prosthetic teeth are easily abraded or corroded over time and create an aesthetic problem such that the whiteness is noticeable in comparison with natural teeth. What is known as material expected to solve these issues is, for example, zirconia. It is possible to manufacture prosthetic teeth with zirconia creating no sense of discomfort when placed side by side with natural teeth.

However, zirconia is originally milky white and prosthetic restoration or restored parts look unnatural when they are not coated. To aesthetically restore teeth, gradation is required for prosthetic tooth to meet the situation of a patient.

In addition, to manufacture a prosthetic tooth with zirconia, it requires molding processing to a desired shape. Therefore, even in the case of cutting with CAD/CAM, experts such as dental technicians cut and color the prosthetic tooth, which is time consuming US2004251574, US2005079086, US2005049739 and US6821462 disclose methods to produce solid freeform fabrication objects, whereby a binder is ejected onto a powdered build material.

### SUMMARY

The invention provides a solid freeform fabrication material set according to claim 1, a method of manufacturing using the material set according to claim 10.

### BRIEF DESCRIPTION OF THE SEVERAL VIEWS OF THE DRAWINGS

Various other objects, features and attendant advantages of the present invention will be more fully appreciated as the same becomes better understood from the detailed description when considered in connection with the accompanying drawings in which like reference characters designate like corresponding parts throughout and wherein:
FIG. 1 is a schematic diagram illustrating an example of the device for manufacturing a solid freeform fabrication object (three-dimensional object) according to an embodiment of the present invention; and
FIG. 2 is a schematic diagram illustrating another example of the device for fabricating a solid freeform object according to an embodiment of the present invention.

### DESCRIPTION OF THE EMBODIMENTS

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

As used herein, the singular forms "a", "an", and "the" are intended to include the plural forms as well, unless the context clearly indicates otherwise.

Moreover, image forming, recording, printing, modeling, etc. in the present disclosure represent the same meaning, unless otherwise specified.

### Solid Freeform Fabrication Material Set

The solid freeform fabrication material set (also referred to as additive manufacturing material set) of the present disclosure includes a first solid freeform fabrication liquid material (also referred to as first additive manufacturing liquid material or slurry) including a solvent, a first resin (referred to herein as resin A) soluble in the solvent, and an inorganic particulate material (referred to herein as "inorganic particle"), and a second solid freeform fabrication liquid material (also referred to as a second additive manufacturing liquid material) including a second resin (referred to herein as resin B) soluble in water and a colorant. The solid freeform fabrication material set also includes other optional materials.

The solid freeform fabrication material set of the present disclosure is provided based on the knowing that, when forming a fabrication object, typically, water is removed to dry the fabrication object so that it is not possible to fill in interlayers with attached binder, resulting in peeling-off and in addition, coloring takes a long time. Moreover, the solid freeform fabrication material set of the present disclosure is also provided based on the knowledge that zirconia before being sintered may excessively absorb colorant in the method of coloring ceramics for dentistry, which makes it difficult to obtain stable coloring and also cutting takes a long time. Furthermore, the solid freeform fabrication material set of the present disclosure is also based on the knowledge that only uniformly-colored blocks are obtained in typical coloring methods so that gradation processing is required and also cutting takes a long time.

The solid freeform fabrication material set of the present disclosure includes the second solid freeform fabrication liquid material including at least one colorant.

In the solid freeform fabrication material set, the second solid freeform fabrication liquid material may include a plurality of colorants. Also, it is possible to include different colorants in the first solid freeform fabrication liquid material and the second solid freeform fabrication liquid material.

Since the first solid freeform fabrication liquid material and the second solid freeform fabrication liquid material include respective resins soluble in respective solvents, the colorants can be disposed in suitable sites after sintering. The resins soluble in the solvents have compatibility with the colorants and the solvents for solid freeform fabrication, which prevents the colorant from being locally present caused by agglomeration, etc. The second solid freeform fabrication liquid material includes the resin B soluble in water.

First Solid Freeform Fabrication Liquid Material (First Additive Manufacturing Liquid Material)

The first solid freeform fabrication liquid material (first additive manufacturing liquid material) includes the solvent, the resin A soluble in the solvent, the inorganic particle, and other optional components.

### Inorganic Particle

The inorganic particle has no specific limit and can be suitably selected to suit to a particular application. For example, ceramic particles and metal particles are usable.

The inorganic particle is preferably biocompatible.

### Ceramic Particle

The ceramic particle has no specific limit and can be suitably selected to suit to a particular application. For example, zirconia particles, alumina particles, silica particles, and lithium disilicate particles are usable. These can be used alone or in combination. Of these, zirconia particles are preferable. When zirconia particles are used as the ceramic particle, it is possible to include yttria, ceria, etc. as a stabilizer.

The volume average particle diameter of the ceramic particle is preferably less than 5 µm and more preferably less than 1 µm in the first solid freeform fabrication liquid material. When the volume average particle diameter is less than 5 µm, density of a green sheet or green body can be prevented form being decreased and it can be suitably sintered to improve mechanical strength. The measuring of the volume average particle diameter of the ceramic particle is not particularly limited and can be measured to suit to a particular application by selecting a known particle size measuring instrument. For example, Multisizer III (manufactured by Beckman Coulter, Inc.) and FPIA-3000 (manufactured by Sysmex Corporation) are used to execute measuring according to a known method.

The green sheet or the green body means a sheet or molded body obtained by injection molding a composition of a kneaded material of a slurry and a resin.

The zirconia particle has an extremely high melting point. Therefore, the volume average particle diameter is decreased before sintering. The ideal volume average particle diameter is several tens nm. If it is 1 µm or greater, a large amount of remaining space between particles makes it difficult to conduct sintering. In order to conduct typical additive manufacturing, it is required to convey material including zirconia particles from a supply tank to a print tank. If the particle constituting the material is small, interparticle force becomes strong, which tends to significantly degrade flowability. Therefore, to improve flowability while maintaining sintering property, it is required to prepare and handle slurry while maintaining the volume average particle diameter under several hundreds nm.

The proportion of the stabilizer (yttria, ceria, etc.) is preferably 2 - 6 percent by mass and more preferably 3 - 5 percent by mass to the total content of the first solid freeform fabrication liquid material. When the proportion is 2 - 6 percent by mass, the stabilizer demonstrates its function to the full and cracking is prevented during sintering.

The content of the stabilizer in the zirconia particle can be measured by, for example, inductively coupled plasma (ICP) atomic emission spectrochemical analysis.

The rate of monoclinic crystal system of the zirconia particle is preferably 30 percent or less and more preferably 20 percent or less. When the rate of monoclinic crystal system is 30 percent or less, tetragonal crystal system becomes suitable so that mechanical strength is good. The rate of monoclinic crystal system of the inorganic particle can be measured by, for example, an X ray powder diffractometer under predetermined conditions.

The method of manufacturing the ceramics particle has no specific limitation and can be suitably selected to suit to a particular application. For example, a thermal decomposition technique, a coprecipitation technique, and a hydrolysis method are suitable.

Of these, the thermal decomposition technique and the coprecipitation technique are preferable for zirconia particles.

In the thermal decomposition technique, for example, a predetermined amount of oxyzirconium chloride and an aqueous solution of yttrium chloride is mixed and the mixture is admixed with sodium chloride (or potassium chloride). The proportion of sodium chloride (or potassium chloride) is 0.1 - 1 percent by mass to the total content of oxyzirconium chloride. This liquid mixture is subjected to instant drying such as spray-drying technique to obtain dried powder.

The instant drying is a technique of drying an article within 10 seconds, which is preferably conducted at 200 degrees C or higher in a heated atmosphere. Next, the dried powder is thermally decomposed in the atmosphere in the temperature range of 800 to 1,200 degrees C to obtain temporarily-baked powder of oxide. The temporarily-baked powder of oxide is pulverized by a wet pulverization method until the pulverization diameter is reduced to 2 µm or less and thereafter washed with water.

There is no specific limitation to the washing method with water. It can be selected to suit to a particular application. For example, a continuous washing filtration method using membrane filters is preferable. The temporarily-baked powder of oxide is sufficiently washed with water in such a manner that the concentration of sodium (or potassium) in the organic particle is reduced to the range of from 0.001 (10 ppm) to 0.01 (100 ppm) percent by mass in oxide conversion. The slurry obtained after the washing with water is dried to obtain inorganic particles (zirconia powder).

In the coprecipitation technique, for example, oxyzirconium chloride and yttrium chloride aqueous solution are mixed. In particular, to form a metal complex to maintain pH at which each hydrate of oxyzirconium chloride and yttrium chloride precipitates, sodium sulfate (or potassium sulfate) is added in such a manner that the molar ratio of sodium sulfate (or potassium sulfate) to zirconia is 0.3 - 0.7 followed by reaction at 50 to 100 degrees C for several hours or more. Alkali aqueous solution of sodium hydroxide, ammonium, etc. is added to this mixture while stirring the aqueous solution to set pH of the solution in the range of 8 to 10.

The thus-obtained coprecipitation hydrate particle is sufficiently washed with water to confirm that sodium (or potassium) is 0.001 (10 ppm) to 0.01 (100 ppm) percent by mass in oxide conversion. The hydrate particulates after washing with water is dehydrated and dried and thereafter baked in the atmosphere at the temperature range of 800 to 1,200 degrees C to obtain temporarily-baked powder of oxide. The thus-obtained temporarily-baked powder of oxide is wet-pulverized to the size of 2 µm or less and dried to obtain inorganic particles (zirconia powder).

### Metal Particle

The metal particle has no specific limit and can be suitably selected to a particular application. For example, titanium particles, particles of alloyed titanium, particles of alloy of cobalt and chrome, and particles of alloyed stainless steel are usable. These can be used alone or in combination. Of these, titanium particles and particles of alloyed titanium are preferable.

The volume average particle diameter of the metal particle is preferably less than 50 µm and more preferably less than 10 µm. When the volume average particle diameter is less than 50 µm, space between particles can be reduced and the density of a green sheet or a green body can be increased. Therefore, the baking shrinking during sintering is reduced, thereby improving the dimension accuracy. The measuring of the volume average particle diameter of the metal particle is not particularly limited and can be measured to suit to a particular application by selecting a known particle size measuring instrument. For example, Multisizer III (manufactured by Beckman Coulter, Inc.) and FPIA-3000 (manufactured by Sysmex Corporation) are used to execute measuring according to known method.

The content ratio of the inorganic material is 20 to 70 parts by mass to 100 parts of the first solid freeform fabrication liquid material (slurry). When the content is 20 parts by mass or greater, the content of vaporized solvent is relatively small and the density of the green sheet or green body can be increased. When the content is 70 parts by mass or less, the flowability as the slurry is improved so that the slurry can be suitably conveyed by a doctor blade, etc.

### Resin A

The resin A has no particular limitation and can be suitably selected to suit to a particular application. "Soluble" in "the resin soluble in the solvent" means that 10 percent by mass or greater is dissolved in the solvent at room temperature (25 degrees C).

The resin A preferably has an acidic functional group reactive with a basic functional group.

Specific examples of the acidic functional group include, but are not limited to, carboxyl group and hydroxyl group.

Examples of the resin A having an acidic functional group are modified polyvinyl alcohol, polyacrylic acid, and polyethylene oxide. These can be used alone or in combination. Of these, polyacrylic acid is preferable in terms of the reactivity with a basic functional group.

The mass average molecular weight (Mw) of the polyacrylic acid is preferably from 400,000 or greater, more preferably from 400,000 to 1,000,000, and furthermore preferably from 600,000 to 800,000. When the mass average molecular weight (Mw) is 400,000 or greater, cross-linking with the resin B having a basic functional group in the second solid freeform fabrication liquid material is easy and curing time for a solid freeform fabrication object is suitable. On the other hand, when the mass average molecular weight (Mw) is 1,000,000 or less, an obtained slurry has a suitable viscosity and the inorganic particles tends to be homogenously present in the slurry. With regard to the mass average molecular weight (Mw), for example, it can be calculated based on the molecular weight distribution of isolated polyacrylic acid obtained by gel permeation chromatography (GPC).

The volume average particle diameter of the resin A has no particular limit and can be suitably selected to suit to a particular application. For example, the resin A preferably has a volume average particle diameter of from 10 to 200 µm. The measuring of the volume average particle diameter is not particularly limited and can be measured by selecting a known particle size measuring instrument to suit to a particular application. For example, Multisizer III (manufactured by Beckman Coulter, Inc.) and FPIA-3000 (manufactured by Sysmex Corporation) are used to execute measuring according to known methods.

The proportion of the resin A is preferably 5 to 30 parts by mass to 100 parts by mass of the inorganic particle. When the content is 5 parts by mass or greater, binding is sufficient. Also, the inorganic particle and the colorant are suitably dispersed in a slurry so that dispersion stability can be improved. When the content is 30 parts by weight or less, viscosity of the slurry can be lowered and the slurry can be suitably conveyed by a doctor blade, etc. The measuring of the content of the resin A is not particularly limited and can be measured by selecting a known thermal analysis instrument to suit to a particular application. For example, DSC-200 (manufactured by Seiko Instruments Inc.) can be used to execute measuring according to known methods.

### Solvent

The solvent has no particular limit and can be suitably selected to suit to a particular application as long as it can dissolve the resin A. For example, polar solvents such as water, methanol, ethanol, and toluene (boiling point: 110.6 degrees C) are preferable. These can be used alone or in combination. Of these, in terms of improving productivity of fabricating a green sheet or green body, organic solvents having low boiling points are preferable.

Organic solvents having boiling points of 80 degrees C or lower are more preferable and ethanol are particularly preferable.

Specific examples of the organic solvents having boiling points of 80 degrees C or lower include, but are not limited to, ethanol (boiling point: 78.37 degrees C), methanol (boiling point: 64.7 degrees C), ethyl acetate (boiling point: 77.1 degrees C), acetone (boiling point: 56 degrees C), and methylene chloride (boiling point: 39.6 degrees C).

### Other Components

The other optional components are not particularly limited and can be suitably selected to suit to a particular application. Examples thereof are colorant, dispersant, plasticizer, and a sintering helping agent.

The colorant has no particular limit and can be selected to suit to a particular application.

For example, the same colorant as those in the second solid freeform fabrication liquid material can be used.

It is preferable that the first solid freeform fabrication liquid material include a dispersant because dispersibility of the inorganic particle is improved and sedimentation during rest can be suppressed. Also, the inorganic particle tends to be continuously present when fabricating a green sheet or a green body. In addition, it is preferable that the first solid freeform fabrication liquid material include a plasticizer because a green sheet or green body formed of the first solid freeform fabrication liquid material tends to be free of cracking when dried. Inclusion of a sintering helping agent is also preferable because sintering at lower temperatures is possible during sintering treatment for an obtained additive manufacturing object.

Second Solid Freeform Fabrication Liquid Material (Second Additive Manufacturing Liquid Material)

The second solid freeform fabrication liquid material (second additive manufacturing liquid material) includes the resin B soluble in water, colorant, and other optional components.

### Resin B

The resin B soluble in water has no specific limit and is suitably selected to suit to a particular application. For example, water-soluble resins are usable. "Soluble" in "the resin soluble in water" means that 10 percent by mass or greater is dissolved in water at room temperature (25 degrees C).

Examples of the water-soluble resin are polyvinyl alcohol having a saponification molar ratio of 98 mole percent or less and polyoxyethylene oxide. These can be used alone or in combination.

In terms of obtaining a solid freeform fabrication object having a high mechanical strength, the resin B is preferably reactive with the resin A contained in the first solid freeform fabrication liquid material.

The resin B preferably has a basic functional group reactive with an acidic functional group.

A specific example of the basic functional group is amino group.

Examples of the resin B having an amino group are polyethylene imine and polyvinyl pyrrolidone. These can be used alone or in combination. Of these, in terms of reactivity with an acidic functional group, polyethylene imine having a high cation density is preferable.

The mass average molecular weight (Mw) of the polyethylene imine is preferably 1,800 or greater, more preferably from 1,800 to 70,000, and furthermore preferably from 3,000 to 20,000. When the mass average molecular weight (Mw) is 1,800 or greater, cross-linking with the resin A having an acidic functional group in the first solid freeform fabrication liquid material is easy and curing time for a solid freeform fabrication object is suitable. When the mass average molecular weight (Mw) is 70,000 or less, the second solid freeform fabrication liquid material has a suitable viscosity and can be stably discharged.

The mass average molecular weight (Mw) can be measured by, for example, gel permeation chromatography (GPC).

The content ratio of the resin B is 3 to 20 parts by mass to 100 parts of the second solid freeform fabrication liquid material. When the content is 3 parts by mass or greater, cross-linking with the resin A in the first solid freeform fabrication liquid material is sufficient and strength of an obtained green sheet or green body can be enhanced. On the other hand, when the content is 20 parts by mass or less, viscosity of the second solid freeform fabrication liquid material can be lowered and discharging stability can be improved.

The measuring of the content of the resin B is not particularly limited and can be measured by selecting a known thermal analysis instrument to suit to a particular application. For example, DSC-200 (manufactured by Seiko Instruments Inc.) can be used to execute measuring according to known methods.

### Colorant

The colorant can be added to control the color of the entirety of an object.

The colorant preferably colors inorganic particle such as zirconia.

As the method of coloring the inorganic particle such as zirconia, method of adding an inorganic colorant followed by sintering and a method of admixing an inorganic particle with an oxide of rare earth metal or transition metal followed by sintering are suitable. Rare earth ions having an absence in f orbital or transition metal ions having a cleaved energy level due to the impact of ligand field while having an absence in d orbital are preferably used because inorganic particles can be colored by a minor quantity in comparison with addition of inorganic pigments.

The rare earth ions or transition metal ions have no specific limit and can be suitably selected to suit to a particular application. For example, ions such as Ce, Pr, Eu, Er, Fe, Co, Ni, Ti, V, Cr, Cu, Mn, Zn, Zr, and Cd are preferable. These can be used alone or in combination. Of these, oxides having, for example, Pr, Fe, Ti, or Zr ion are preferable in terms of biocompatibility.

In addition to the oxides, metal salts having negative ions such as Cl⁻, SO₄⁻, SO₃⁻, Br⁻, F⁻, NO₂⁻, and NO₃⁻ are usable.

Synthesized material or products available on the market can be the colorant. Specific examples of the products available on the market include, but are not limited to, P-40 (Zr-Si-Pr-Fe based, manufactured by NITTO GANRYO KOGYO CO., LTD.) and M-67 (Zr-Si-Pr-Fe based, manufactured by NITTO GANRYO KOGYO CO., LTD.).

Natural teeth are colored yellow to brown. For example, the optical values of A1 to A4 of Vita Shade Guide (hereinafter referred to as Guide, created by Hakusui Trading CO., LTD.) are in the range of from L*: +75 to +55, a*: -10 to +10, b*: +5 to +25). Therefore, a colored solid freeform fabrication object colored by the colorant preferably has color space suiting to the color of a natural tooth. Since metal does not look like a natural tooth, a case in which pigments are contained is described. Specifically, L*, a*, and b* of a solid freeform fabrication object are preferably within the following range.

L* is preferably +95 or less, more preferably +85 to +50, and particularly preferably from +75 to +55.

a* is preferably +40 to -10 as ceramic particle and preferably +60 to +10 as metal.

b* is preferably -10 or greater and more preferably from +15 to +35. a* and b* have different color spaces depending on the type in the Guide. The following description is about the type A in the Guide. a* obtained according to the type A in the Guide is preferably +10 to -10 as ceramic particle and preferably +30 to +10 as metal. b* is +5 or greater and more preferably +15 to +35.

Inkjet methods are preferable to place the colorant in suitable positions

The colorant can be added to the first solid freeform fabrication liquid material in addition to the second solid freeform fabrication liquid material. It is efficient to add a colorant to the first solid freeform fabrication liquid material because the colorant can be uniformly applied to the entire surface of the fabrication area, preventing color unevenness. However, it is not possible to apply a colorant to an arbitrary site to add gradation. For this reason, since the colorant is contained in the second solid freeform fabrication liquid material, a suitable amount of a suitable kind of colorant can be applied to arbitrary sites so that gradation can be applied during fabrication. As a result, more value addition can be expected.

The application of colorant can be controlled by applying a colorant at a particular site in a desired amount. That is, the amount of the colorant in the first solid freeform fabrication liquid material and the second solid freeform fabrication liquid material is not a question. However, if the colorant is contained in the first solid freeform fabrication liquid material, the representable color space is reduced and the colorant agglomerates by an external force (for example, dispersion using a medium) added to stabilize the inorganic particle in a liquid. Therefore, the content (percent by mass) of the colorant in the second solid freeform fabrication liquid material is preferably greater than the content (percent by mass) of the colorant in the first solid freeform fabrication liquid material.

The proportion of the colorant is preferably 10 percent by mass or less in terms of inkjet discharging and 1.0 to 5.0 percent by mass in terms of coloring and inkjet dischargeability to the entire amount of the second solid freeform fabrication liquid material.

The content of the colorant is preferably 0.01 to 5 parts by mass to 100 parts by mass of the first solid freeform fabrication liquid material. When the content is 0.01 to 5 parts by mass, an increase of viscosity and non-uniformity of compositions can be prevented and smooth layers can be formed.

When the total content of the colorant contained in the first solid freeform fabrication liquid material and the second solid freeform fabrication liquid material is 0.01 to 5 parts by mass to 100 parts by mass of the inorganic particle in the first solid freeform fabrication liquid material, color tone and half transparency can be imparted.

Also, it is preferable that the mass ratio of the colorant in the second solid freeform fabrication liquid material be higher than the mass ratio of the colorant in the first second solid freeform fabrication liquid material.

### Other Components

As the other components, for example, aqueous medium, surfactants, preservatives, anti-septic agents, stabilizers, and pH regulators are suitable.

### Aqueous Medium

Water or a mixture of water and an organic solvent can be the aqueous medium. The organic solvent is, for example, alcohols such as methanol and ethanol, ethers, and ketones. These can be used alone or in combination. It is preferable that no phase separation occur when mixed with water. Of these, water is preferable.

As the water, deionized water, ultrafiltered water, reverse osmosis water, pure water such as distilled water, and ultra pure water are suitable.

The second solid freeform fabrication liquid material can be applied to simple and efficient manufacturing of various additive manufacturing objects and structures and also particularly suitably applied to the method of manufacturing a solid freeform fabrication object of the present disclosure and the device for manufacturing a solid freeform fabrication object of the present disclosure.

### Method of Manufacturing Solid Freeform Fabrication Object (Additive Manufacturing Object) and Device for Manufacturing Solid Freeform Fabrication Object (Additive Manufacturing Object)

The method of manufacturing a solid freeform fabrication object (additive manufacturing object) of the present disclosure includes layer forming and liquid material application, preferably layer drying and sintering, and other optional processes.

The device for manufacturing a solid freeform object (additive manufacturing object) of the present disclosure includes a layer-forming device, a liquid material application device, preferably a layer drying device and a sintering device, and other optional devices.

The method of manufacturing a solid freeform object (additive manufacturing object) of the present disclosure can be suitably executed by the device for manufacturing a solid freeform object of the present disclosure. The layer forming can be suitably conducted by the layer forming device and the liquid material application is suitably conducted by the liquid material applying device. The sintering can be executed by the sintering device. The other optional processes can be executed by the other optional devices.

### Layer Forming Process and Layer Forming Device

In the layer forming process, the first solid freeform fabrication liquid material layer is formed by using the first solid freeform fabrication liquid material.

The layer forming device forms the first solid freeform fabrication liquid material layer by using the first solid freeform fabrication liquid material.

### Substrate

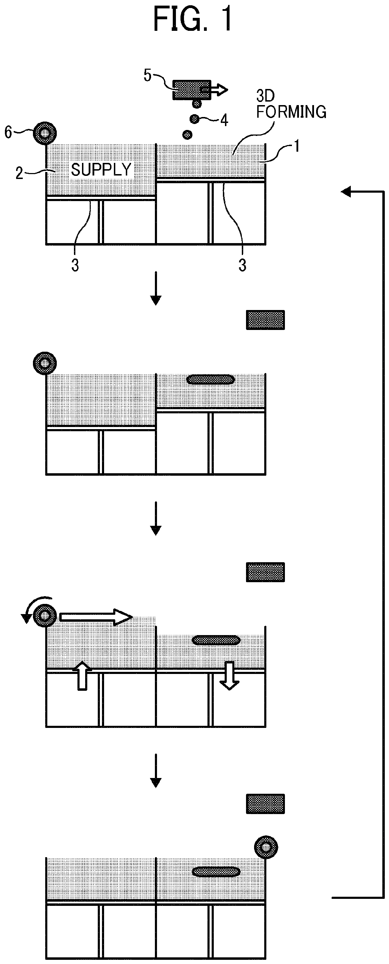

The substrate (liquid material layer holding device) on which the first solid freeform fabrication liquid material can be placed is not particularly limited and can be suitably selected to suit to a particular application. For example, a plate having a surface to place the first solid freeform fabrication liquid material on and a base plate having a surface illustrated in FIG. 1 of Japanese Unexamined Patent Application Publication No. 2000-328106 are suitable. The surface of the substrate, that is, the surface on which the first solid freeform fabrication liquid material is placed can be smooth, coarse, plane, or curved.

### Forming First Solid Freeform Fabrication Liquid Material Layer

The method of placing the first solid freeform fabrication liquid material on the substrate is not particularly limited and can be suitably selected to suit to a particular application. For example, a method of using a known counter rotation mechanism (counter roller) for use in a selective laser sintering method disclosed in Japanese Translation of PCT International Publication No. JP-T-2000-505737A, a method of extending the slurry material to a thin layer using a member such as a brush, a roller, and a blade, a method of pressing the surface of the slurry material layer using a pressure member to extend to a thin layer, and a method of using a known powder additive manufacturing device are suitable as the method of placing the first solid freeform fabrication liquid material (slurry material) as a thin layer.

Using the counter rotation mechanism (counter roller), the brush, the blade, or the pressing member, the slurry material can be placed on the substrate, for example, in the following manner: That is, in an outer frame (also referred to as "mold", "hollow cylinder", "tubular structure", etc.), the slurry material is placed onto the substrate disposed slidably movable up and down along the inside wall of the outer frame by the counter rotation mechanism (counter roller), the brush, the roller, the blade, the pressing member, etc. At this point, if, as the substrate, a substrate movable up and down in the outer frame is used, the substrate is arranged slightly lower than the upper opening of the outer frame. That is, the slurry material is caused to be placed on the substrate while the substrate is placed lower than the upper hole with an amount corresponding to the layer thickness of the first solid freeform fabrication liquid material (slurry material). As a result, a thin layer of the slurry material can be placed on the substrate.

When laser beams, electron beams, or the second solid freeform fabrication liquid material is applied or supplied to the thin layer of the slurry material, the thin layer is cured. Thereafter, if the slurry material is placed on the cured thin layer in the same manner as described above and laser beams, electron beams, or the second solid freeform fabrication liquid material is applied to the slurry material (layer) placed on the thin layer, curing occurs. That curing occurs not only at the slurry material (layer) placed on the thin layer but also the border between the cured material layer and the previously cured thin layer present below the cured material layer.

As a consequence, the cured object (solid freeform fabrication object) is obtained having a thickness corresponding to approximately the two layers of the slurry material (layer) placed on the previously-placed thin layer.

In addition, it is possible to automatically and simply place a thin layer of the slurry material on the substrate mentioned above by using the known powder additive manufacturing device mentioned above. Typically, such a powder additive manufacturing device has a recoater to laminate the slurry material, a movable supplying tank to supply the slurry material onto the substrate mentioned above, and a movable forming tank to place thin layers of the powder material to laminate the thin layers. In the powder additive manufacturing device, the supplying tank is elevated, the forming tank is elevated down, or both to set the surface of the supplying tank slightly above the surface of the forming tank. In addition, the powder material is arranged to form a thin layer using the recoater from the side of the supplying tank and the recoater is repetitively moved to laminate the thin layers of the powder material. This powder material additive manufacturing device can be entirely replaced with a device for slurry additive manufacturing or the recoater portion can be substituted with a doctor blade for sheet forming.

The thickness of the slurry material layer is not particularly limited and can be selected to suit to a particular application. For example, the average thickness for a single layer is preferably 3 to 200 µm and more preferably 10 to 100 µm. When the average thickness is 3 µm or greater, the time to be taken to obtain a solid freeform fabrication object can be appropriate and problems such as shape losing does not occur during processing such as sintering and/or handling. When the average thickness is 200 µm or less, uniform coloring and dimension accuracy of a solid freeform fabrication object is sufficiently obtained. The average thickness can be measured according to a known method.

### Layer Drying Process and Layer Drying Device

In the layer drying process, the obtained slurry layer is dried before the liquid material applying process and after the layer forming process. The layer drying process is conducted by a layer drying device. Natural drying is also possible. In the layer drying process, it is possible to evaporate the solvent contained in the slurry layer. In the layer drying process, it is preferable that not all the solvent be removed from the slurry layer to obtain a half-dried state. As the layer drying device, for example, known driers can be used.

The drying time in the layer drying process can be appropriately changed. If the drying time is long, it is possible to suppress oozing in the horizontal direction of the liquid material applied in the liquid material applying process after the layer drying process, which leads to improvement of fabrication accuracy. However, attachment force between layers tends to deteriorate. If the drying time is shortened, particles move between layers so that attachment force between layers is strengthened. However, oozing in the horizontal direction of the liquid material applied in the liquid material applying process after the layer drying process occurs, which leads to degradation of fabrication accuracy. It is suitable to determine the drying time depending on the material used.

### Liquid Material Applying Process and Liquid Material Applying Device

In the liquid material applying process, the second solid freeform fabrication liquid material (additive manufacturing material) containing an aqueous medium is applied to a predetermined area of the first solid freeform fabrication liquid material layer.

The liquid material applying device applies the second solid freeform fabrication liquid material (additive manufacturing material) containing an aqueous medium to the predetermined area of the first solid freeform fabrication liquid material layer.

The method of applying the second solid freeform fabrication liquid material to the slurry material layer has no particular limit and can be suitably selected to suit to a particular application. For example, the method of applying the second solid freeform fabrication liquid material is executed by a liquid discharging device used in dispenser methods, spray methods, or inkjet methods. In the present disclosure, the liquid discharging device for use in the inkjet method (which uses an oscillator such as a piezoelectric actuator to discharge droplets from multiple nozzles) is preferable in terms that complex solid freeform objects can be efficiently and precisely fabricated.

### Removing Process and Removing Device

In the removing process, uncured slurry material is removed from an area other than the predetermined area mentioned above, where no second solid freeform fabrication liquid material is applied after sequentially repeating the layer forming and the liquid material application. This removal is preferably conducted by liquid immersion.

The removing device immerses a solid freeform fabrication object formed by sequentially repeating the layer forming process and the liquid material applying process in liquid to remove uncured slurry material.

An example of the liquid is water.

### Sintering Process and Sintering Device

A solid freeform fabrication object (additive manufacturing object) formed by sequentially repeating the layer forming process and the liquid material applying process is sintered in the sintering process, which is executed by a sintering device. According to the sintering process, the cured material is formed as an integrated object (sintered compact).

For example, a known sintering furnace can be used as the sintering device.

When the sintering temperature is 1,350 to 1,600 degrees C, a sintered compact having good coloring and strength is obtained.

In addition to the method of sintering a cured object as described above, sintering can be conducted when laminating the first solid freeform fabrication liquid material. The method of conducting sintering at the time of laminating the first solid freeform fabrication liquid material includes irradiating the first solid freeform fabrication liquid material layer with a laser beam or an electron beam to sinter the first solid freeform fabrication liquid material layer.

### Irradiation of Laser

The laser for use in the laser irradiation has no particular limit and can be suitably selected to suit to a particular application. For example, CO₂ laser, Nd-YAG laser, fiber laser, and semi-conductor laser can be used. There is no specific limitation to the condition of laser irradiation. It can be suitably selected to suit to a particular application. For example, when a small-sized laser is used, it is not possible to melt the powder material mentioned above. Therefore, it is preferable that an adhesive (e.g., polyester-based additive) be mixed and melted by laser irradiation to fabricate an object. In such a case, CO₂ laser is preferable. As the irradiation conditions, for example, it is preferable that the laser power be 15 W, the wavelength be 10.6 µm and the beam diameter be about 0.4 mm.

### Irradiation of Electron Beam

There is no specific limitation to the electron beams if the beams melt the inorganic particle in the first solid freeform fabrication liquid material. It can be selected to suit to a particular application. When the first solid freeform fabrication liquid material is irradiated with an electron beam, the material is required to be placed in a vacuum condition. There is no specific limitation to the conditions of irradiation of electron beams. It can be suitably selected to suit to a particular application. For example, it is preferable that the laser power be 1,500 W, the beam diameter be about 0.1 mm, and the vacuum degree be about 1.0 × 10⁻⁵ mbar.

### Other Processes and Other Devices

The other processes include a surface protection treatment process, a coating (application) process, etc.

The other devices include a surface protection treatment device, a coating device, etc.

### Surface Protection Process and Surface Protection Device

The surface protection treatment process is to form a protection layer on an object formed in the liquid application process or the sintering process. Due to the surface protection treatment process, durability is imparted to the surface of the solid freeform object to the degree that, for example, the object can be used as is.

Specific examples of the protection layer include, but are not limited to, a water-resistance layer, a weather resistance layer, a light resistance layer, a heat insulation layer, and a gloss layer.

Specific examples of the surface protection treatment device include, but are not limited to, known surface protection treatment devices such as a spraying device and a coating device.

### Application Process and Application Device

The coating process is to coat the solid freeform fabrication object. Due to this coating process, the color obtained for the solid freeform object can be corrected to a desired color. Specific examples of the coating device include, but are not limited to, known coating devices using a spray, a roller, a brush, etc.

FIG. 1 is a diagram illustrating an example of the device for manufacturing a solid freeform object for use in the present disclosure. The device for manufacturing a solid freeform object illustrated in FIG. 1 includes a slurry storage tank 1 on the fabrication side and a slurry storage tank 2 on the supply side, each of which includes a stage 3 movable up and down and forms layers formed of slurry material on the stage 3.

The device for fabricating a solid freeform object has an inkjet head 5 over the slurry storage tank 1 to discharge a second solid freeform fabrication liquid material 4 toward the first solid freeform fabrication liquid material (slurry material) in the slurry storage tank 1. Moreover, the device for fabricating a solid freeform fabrication also includes a smoothing mechanism (hereinafter also referred to as recoater) 6 to supply the slurry material from the slurry storage tank 2 to the slurry storage tank 1 and smooth the surface of the layer of the slurry material in the slurry storage tank 1.

The second solid freeform fabrication liquid material 4 is dripped from the inkjet head 5 onto the slurry material in the slurry storage tank 1. During the dripping, the position where the second solid freeform fabrication liquid material 4 is dripped is determined by two-dimensional image data (slice data) obtained by slicing a target 3D shape into multiple plane layers.

After completing depiction in an amount corresponding to a single layer, the stage 3 for the slurry storage tank 2 is elevated while the stage 3 of the slurry tank 1 is elevated down. The amount of the slurry material corresponding to the difference is moved to the slurry storage tank 1 by the smoothing mechanism 6.

This is how a new layer of the slurry material is formed on the surface of the previously formed layer of the slurry material. The thickness per layer of the slurry material is about several tens µm to about several hundreds µm. Furthermore, depiction is conducted on the newly-formed slurry layer based on slice data for the second layer. A series of these processes are repeated to obtain a solid freeform fabrication object. Subsequent to heating and drying by a heater, a final fabrication object is obtained.

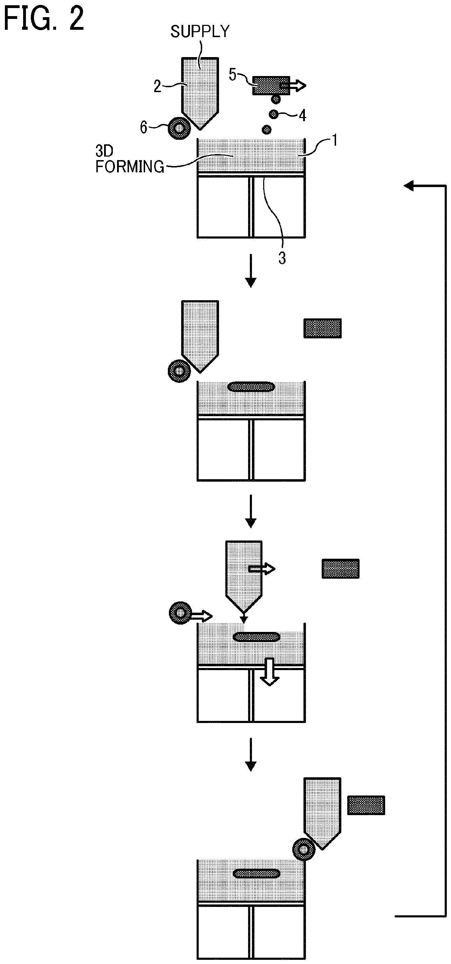

FIG. 2 is a diagram illustrating another example of the slurry additive manufacturing device for use in the present disclosure. The method of manufacturing a solid freeform fabrication object illustrated in FIG. 2 is executed on the same principle as that illustrated in FIG. 1. However both have different supplying mechanisms for the first solid freeform fabrication liquid material (slurry material). That is, the slurry storage tank 2 is disposed above the slurry storage tank 1. When the depiction of the first layer is finished, the stage 3 of the slurry storage tank 1 is lowered in a predetermined amount and the slurry material is dropped from the slurry storage tank 2 to the slurry storage tank 1 while moving the slurry storage tank 2 in order to form a new layer of the slurry material. Thereafter, the recoater 6 compresses the layer of the first solid freeform fabrication liquid material (slurry material) to increase the bulk density and smooths the height of the layer of the slurry material.

According to the device for manufacturing a solid freeform fabrication object illustrated in FIG. 2, the configuration of the device can be compact in comparison with the configuration illustrated in FIG. 1 in which the two slurry storage tanks are disposed in two dimensional manner.

### Dental Prosthesis

Dental prostheses can be manufactured by the method of manufacturing a solid freeform fabrication object of the present disclosure.

As the dental prosthesis, artificial teeth are preferable because the artificial teeth can stand manducation in mouth for a long period of time and has sensuousness.

The artificial teeth are made to restore the function of natural teeth which is lost due to caries, wound, gum diseases, etc. The artificial teeth include bridges and crowns.

According to the method and the device for manufacturing a solid freeform fabrication object of the present disclosure, complex solid freeform fabrication objects in which non-white colorant is appropriately disposed can be simply and stably manufactured using a material having a high melting point and high hardness. In the thus-obtained solid freeform fabrication object (cured object), since a suitable amount of a suitable kind of colorant is applied at desired sites, gradation can be added during fabrication. For this reason, value-added fabrication is possible such as appearances like natural teeth and appearances with good design. In addition, it is possible to suppress change of color caused by abrasion by applying color to the inside of a cured object. Moreover, since the thus-obtained solid freeform fabrication object is free of cell toxicity, has good strength and good dimension accuracy, and reproduces fine roughness and curved planes, the object has good quality so that it can be applied in various fields.

Having generally described preferred embodiments of this invention, further understanding can be obtained by reference to certain specific examples which are provided herein for the purpose of illustration only and are not intended to be limiting. In the descriptions in the following examples, the numbers represent weight ratios in parts, unless otherwise specified.

### EXAMPLES

Next, embodiments of the present disclosure are described in detail with reference to Examples but not limited thereto.

The volume average particle diameter of the inorganic particle in the solid freefrom fabrication liquid material was measured in the following manner.

### Volume Average Particle Diameter of Inorganic Particle

The volume average particle diameter of the inorganic particle in the first solid freeform fabrication liquid material (slurry material) was measured by using LA-920 (manufactured by HORIBA, Ltd.) When measuring with LA-920, application (ver 3.32, created by HORIBA, Ltd.) specialized for LA-920 was used for analysis. Specifically, optical axes were adjusted by chloroform to measure the back ground. Thereafter, circulation was started and the first solid freeform fabrication liquid material (slurry material) 1 was dripped. After checking that the transmittance was stabilized, ultrasonic wave was emitted under the following conditions. After the irradiation, the volume average particle diameter was measured in the transmittance range of from 70 to 95 percent.

In terms of measuring reproducibility of the volume average particle diameter, it was measured under the condition that the transmittance of LA-920 was 70 to 95 percent. In addition, when the transmittance was outside the range mentioned above after the emission of ultrasonic wave, the volume average particle diameter was measured again. To obtain the transmittance within the range, the amount of dripping the first solid freeform fabrication liquid material (slurry material) was controlled. The measuring and analysis conditions were as follows.

**Measuring and Analysis Condition**

| | |
|---|---|
| • Number of data acquisition: | 15 times |
| • Relative refractive index: | 1.20 |
| • Circulation: | 5 |
| • Ultrasonic wave strength: | 7 |

### Synthesis of Inorganic Particle 1

An aqueous solution of 18 percent by mass yttrium chloride was mixed with an aqueous solution of 20 percent by mass oxyzirconia chloride in such a manner that the molar conversion ratio of yttrium and zirconia was 2.8 : 97.2. To the mixture, 0.5 percent by mass of sodium chloride was dissolved to the total content of oxyzirconia chloride to obtain an aqueous solution.

Thereafter, aluminum chloride was added to and dissolved in the obtained aqueous solution in such a manner that alumina accounted for 0.4 percent by mass of the total content of zirconia. This aqueous solution was sprayed and dried in the atmosphere at 200 degrees C to obtain dried powder. The thus-obtained dried powder was baked in the atmosphere at 1,000 degrees C to synthesize temporarily-baked powder. This temporarily-baked powder was pulverized by a wet attritor to obtain 30 percent by mass slurry. Thereafter, the thus-obtained slurry was subject to condensation by repeated dilution and filtration with a membrane filter having an opening of 0.5 µm and thereafter washed until the electric conductivity of the filtrate water was 20 µs or less to synthesize an inorganic particle 1 (zirconia particle).

### Preparation Example or Resin 1

Epoxy resin powder (TORAYPEARL™ EP, manufactured by Toray Industries, Inc.) was subject to dry-classification twice using Turboplex (manufactured by Hosokawa Micron Corporation) to prepare a resin 1 (epoxy resin). The yield was 11 percent by mass and the volume average particle diameter of the resin 1 was 16.8 µm. The volume average particle diameter (Dv) of the resin 1 was measured by Multisizer II (manufactured by Beckman Coulter Inc.).

The thus-prepared resin 1 was immersed in a solvent (ethanol) at room temperature (25 degrees C) but little or never dissolved.

### Preparation Example 1 of First Solid Free form Fabrication Liquid Material (Slurry Material)

### Preparation of First Solid Free form Fabrication Liquid Material (Slurry Material) 1

The following recipe was mixed and dispersed by a bead mill with zirconia beads having a diameter of 3 mm for three hours. Thereafter, the resultant was filtrated by membrane filter having an opening of 0.8 µm to obtain a first solid freeform fabrication liquid material 1.

| | |
|---|---|
| • Inorganic particle 1 (zirconia particle) having a mass average molecular weight (Mw) of 80,000: | 30.0 parts |
| • Polyethylene oxide (PEO) (ALKOX L-8, manufactured by Meisei Chemical Works, Ltd.): | 5.0 parts |
| • Benzylbutyl phthalate as plasticizer: | 10.0 parts |
| • Ceramics dispersant (MALIALIM™ AKM-0531, manufactured by NOF CORPORATION): | 1.5 parts |
| • Ethanol: | 53.5 parts |

The volume average particle diameter of the inorganic particle in the first solid freeform fabrication liquid material 1 was 0.15 µm.

### Preparation Example 2 of First Solid Free form Fabrication Liquid Material (Slurry Material)

### Preparation of First Solid Free form Fabrication Liquid Material (Slurry Material) 2

The following recipe was mixed and dispersed by a bead mill with zirconia beads having a diameter of 3 mm for three hours. Thereafter, the resultant was filtrated by membrane filter having an opening of 0.8 µm to obtain a first solid freeform fabrication liquid material 2.

| | |
|---|---|
| • Inorganic particle 1 (zirconia particle): | 30.0 parts |
| • Benzylbutyl phthalate: | 10.0 parts |
| • Ceramics dispersant (MALIALIM™ AKM-0531, manufactured by NOF CORPORATION): | 1.5 parts |
| • Ethanol: | 58.5 parts |

The volume average particle diameter of the inorganic particle in the first solid freeform fabrication liquid material 2 was 0.15 µm.

### Preparation Example 3 of First Solid Free form Fabrication Liquid Material (Slurry Material)

### Preparation of First Solid Free form Fabrication Liquid Material (Slurry Material) 3

The following recipe was mixed and dispersed by a bead mill with zirconia beads having a diameter of 3 mm for three hours. Thereafter, the resultant was filtrated by membrane filter having an opening of 0.8 µm to obtain a first solid freeform fabrication liquid material 3.

| | |
|---|---|
| • Inorganic particle 1 (zirconia particle): | 30.0 parts |
| • Resin 1 (epoxy resin): | 5.0 parts |
| • Benzylbutyl phthalate: | 10.0 parts |
| • Ceramics dispersant (MALIALIM™ AKM-0531, manufactured by NOF CORPORATION): | 1.5 parts |
| • Ethanol: | 53.5 parts |

The volume average particle diameter of the inorganic particle in the first solid freeform fabrication liquid material 3 was 0.15 µm.

### Preparation Example 4 of First Solid Free form Fabrication Liquid Material (Slurry Material)

### Preparation of First Solid Free form Fabrication Liquid Material (Slurry Material) 4

The following recipe was mixed and dispersed by a bead mill with zirconia beads having a diameter of 3 mm for three hours. Thereafter, the resultant was filtrated by membrane filter having an opening of 0.8 µm to obtain a first solid freeform fabrication liquid material 4.

| | |
|---|---|
| • Inorganic particle 1 (zirconia particle): | 30.0 parts |
| • Polyethylene oxide (PEO) (ALKOX L-8, manufactured by Meisei Chemical Works, Ltd.): | 5.0 parts |
| • Benzylbutyl phthalate: | 10.0 parts |
| • Ceramics dispersant (MALIALIM™ AKM-0531, manufactured by NOF CORPORATION): | 1.5 parts |
| • Inorganic pigment (P-40, Zr-Si-Pr based pigment, manufactured by NITTO GANRYO KOGYO CO., LTD.): | 1.0 part |
| • Ethanol: | 52.5 parts |

The volume average particle diameter of the inorganic particle in the first solid freeform fabrication liquid material 4 was 0.15 µm.

### Preparation Example 5 of First Solid Free form Fabrication Liquid Material (Slurry Material)

### Preparation of First Solid Free form Fabrication Liquid Material (Slurry Material) 5

The following recipe was mixed and dispersed by a bead mill with zirconia beads having a diameter of 3 mm for three hours to obtain a first solid freeform fabrication liquid material 5.

| | |
|---|---|
| • Inorganic particle 1 (zirconia particle): | 30.0 parts |
| • Polyacrylic acid (PAA) (AS-58, manufactured by Nippon Shokubai Co.,Ltd.): | 5.0 parts |
| • Benzylbutyl phthalate as plasticizer: | 10.0 parts |
| • Ceramics dispersant (MALIALIM™ AKM-0531, manufactured by NOF CORPORATION): | 1.5 parts |
| • Ethanol: | 53.5 parts |

The volume average particle diameter of the inorganic particle in the first solid freeform fabrication liquid material was 0.15 µm.

### Preparation Example 6 of First Solid Free form Fabrication Liquid Material (Slurry Material)

### Preparation of First Solid Free form Fabrication Liquid Material (Slurry Material) 6

A first solid freeform fabrication liquid material 6 was obtained in the same manner as in the Preparation Example 5 of the first solid freeform fabrication liquid material except that the inorganic particle 1 (zirconia particle) was changed to the titanium oxide particle (SA-120, manufactured by SAKAI CHEMICAL INDUSTRY CO., LTD.).

The volume average particle diameter of the inorganic particle in the first solid freeform fabrication liquid material was 0.15 µm.

### Preparation Example 7 of First Solid Free form Fabrication Liquid Material (Slurry Material)

### Preparation of First Solid Free form Fabrication Liquid Material (Slurry Material) 7

The following recipe was mixed and dispersed by a bead mill with zirconia beads having a diameter of 3 mm for three hours. Thereafter, the resultant was filtrated by membrane filter having an opening of 0.8 µm to obtain a first solid freeform fabrication liquid material 7.

| | |
|---|---|
| • Inorganic particle 1 (zirconia particle): | 30.0 parts |
| • Polyacrylic acid (PAA) (AS-58, manufactured by Nippon Shokubai Co., Ltd.) | 5.0 parts |
| • Benzylbutyl phthalate as plasticizer: | 10.0 parts |
| • Ceramics dispersant (MALIALIM™ AKM-0531, manufactured by NOF CORPORATION): | 1.5 parts |
| • Inorganic pigment (P-40, Zr-Si-Pr based pigment, manufactured by NITTO GANRYO KOGYO CO., LTD.): | 1.0 part |
| • Ethanol: | 52.5 parts |

The volume average particle diameter of the inorganic particle in the first solid freeform fabrication liquid material was 0.6 µm.

Compositions of first solid freeform fabrication liquid materials (slurry materials) 1 to 7 are shown in Table 1.

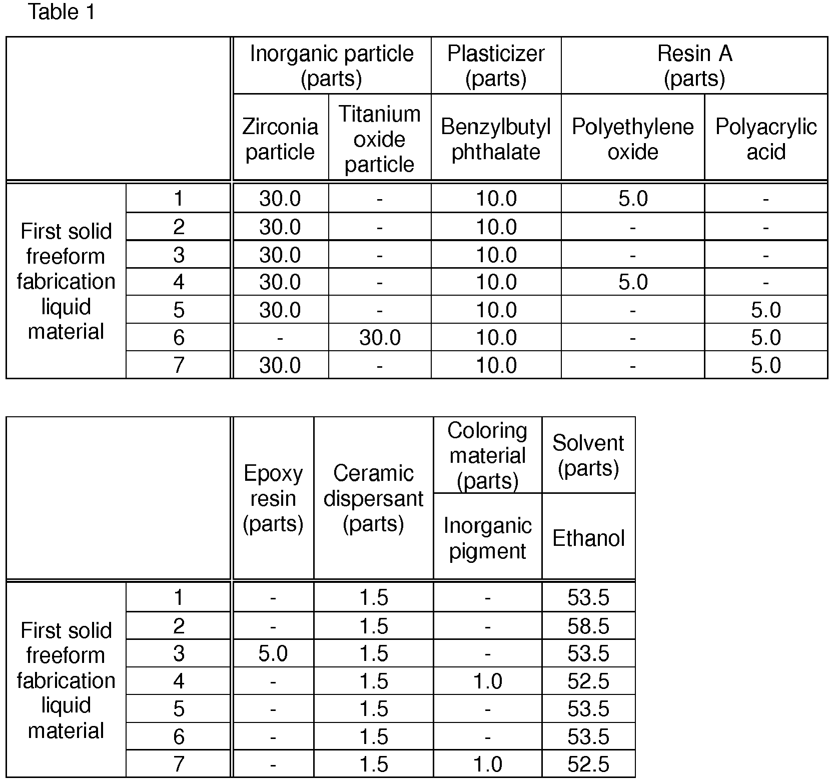

In Table 1, the product name and the manufacturing company of the ingredients are as follows:
- Titanium oxide (TiO₂, SA-120, manufactured by SAKAI CHEMICAL INDUSTRY CO., LTD.)
- Polyethylene oxide (PEO, ALKOX L-8, manufactured by Meisei Chemical Works, Ltd.)
- Polyacrylic acid (PAA) (AS-58, manufactured by Nippon Shokubai Co., Ltd.)
- Ceramic dispersant (MALIALIM™ AKM-0531, manufactured by NOF CORPORATION)
- Inorganic pigment (P-40, Zr-Si-Pr based pigment, manufactured by NITTO GANRYO KOGYO CO., LTD.)

### Preparation Example 1 of Second Solid Freeform Fabrication Liquid Material

### Preparation of Second Solid Freeform Fabrication Liquid Material 1

The following recipe was dispersed by a homomixer for 30 minutes and placed in a mayonnaise bottle in which 15.0 parts of dispersion beads (TORAYCERAM™, manufactured by Tokyo Chemical Industry Co. Ltd.) having a diameter of 1.0 mm were put. After stirring all day, the beads were removed to obtain a second solid freeform fabrication liquid material 1.

| | |
|---|---|
| • Water: | 54.5 parts |
| • Polyethylene oxide (PEO, ALKOX L-8, manufactured by Meisei Chemical Works, Ltd.) having a mass average molecular weight (Mw) of 80,000: | 20.0 parts |
| • Propylene glycol: | 20.0 parts |
| • Inorganic pigment (P-40, Zr-Si-Pr based pigment, manufactured by NITTO GANRYO KOGYO CO., LTD.): | 5.0 parts |
| • Surfactant (Tween 20, manufactured by Tokyo Chemical Industry Co. Ltd.): | 0.5 parts. |

### Preparation Example 2 of Second Solid Freeform Fabrication Liquid Material

### Preparation of Second Solid Freeform Fabrication Liquid Material 2

The following recipe was dispersed by a homomixer for 30 minutes and placed in a mayonnaise bottle in which 15.0 parts of dispersion beads having a diameter of 1.0 mm were put. After stirring all day, the beads were removed to obtain a second solid freeform fabrication liquid material 2.

| | |
|---|---|
| • Water: | 74.5 parts |
| • Propylene glycol: | 20.0 parts |
| • Inorganic pigment (P-40, Zr-Si-Pr based pigment, manufactured by NITTO GANRYO KOGYO CO., LTD.): | 5.0 parts |
| • Surfactant (Tween 20, manufactured by Tokyo Chemical Industry Co. Ltd.): | 0.5 parts. |

### Preparation Example 3 of Second Solid Freeform Fabrication Liquid Material

### Preparation of Second Solid Freeform Fabrication Liquid Material 3

The following recipe was dispersed by a homomixer for 30 minutes and placed in a mayonnaise bottle in which 15.0 parts of dispersion beads having a diameter of 1.0 mm were put. After stirring all day, the beads were removed to obtain a second solid freeform fabrication liquid material 3.

| | |
|---|---|
| • Water: | 72.5 parts |
| • Carboxymethyl cellulose (CMC DAICEL 1350, manufactured by DAICEL FINECHEM LTD.) : | 2.0 parts |
| • Propylene glycol: | 20.0 parts |
| • Inorganic pigment (P-40, Zr-Si-Pr based pigment, manufactured by NITTO GANRYO KOGYO CO., LTD.): | 5 parts |
| • Surfactant (Tween 20, manufactured by Tokyo Chemical Industry Co. Ltd.): | 0.5 parts. |

### Preparation Example 4 of Second Solid Freeform Fabrication Liquid Material

### Preparation of Second Solid Freeform Fabrication Liquid Material 4

A second solid freeform fabrication liquid material 4 was obtained in the same manner as in the Preparation Example 1 of the second solid freeform fabrication liquid material except that the content of water was changed from 74.5 parts to 56.5 parts and the content of the inorganic pigment (P-40) was changed from 5.0 parts to 3.0 parts.

### Preparation Example 5 of Second Solid Freeform Fabrication Liquid Material

### Preparation of Second Solid Freeform Fabrication Liquid Material 5

The following recipe was dispersed by a homomixer for 30 minutes and placed in a mayonnaise bottle in which 15.0 parts of dispersion beads (TORAYCERAM™, manufactured by Tokyo Chemical Industry Co. Ltd.) having a diameter of 1.0 mm were put. After stirring all day, the beads were removed to obtain a second solid freeform fabrication liquid material 5.

| | |
|---|---|
| • Water: | 62.5 parts |
| • Polyethylene imine (PEI) (SP-200, manufactured by Nippon Shokubai Co., Ltd.) having a mass average molecular weight (Mw) of 10,000: | 12.0 parts |
| • Propylene glycol: | 20.0 parts |
| • Inorganic pigment (P-40, Zr-Si-Pr based pigment, manufactured by NITTO GANRYO KOGYO CO., LTD.): | 5.0 parts |
| • Surfactant (Tween 20, manufactured by Tokyo Chemical Industry Co. Ltd.): | 0.5 parts. |

### Preparation Example 6 of Second Solid Freeform Fabrication Liquid Material

### Preparation of Second Solid Freeform Fabrication Liquid Material 6

The following recipe was dispersed by a homomixer for 30 minutes and placed in a mayonnaise bottle in which 15.0 parts of dispersion beads (TORAYCERAM™, manufactured by Tokyo Chemical Industry Co. Ltd.) having a diameter of 1.0 mm were put. After stirring all day, the beads were removed to obtain a second solid freeform fabrication liquid material 6.

| | |
|---|---|
| • Water: | 67.5 parts |
| • Polyethylene imine (PEI, SP-200, manufactured by Nippon Shokubai Co., Ltd.) having a mass average molecular weight (Mw) of 10,000: | 12.0 parts |
| • Propylene glycol: | 20.0 parts |
| • Surfactant (Tween 20, manufactured by Tokyo Chemical Industry Co. Ltd.): | 0.5 parts. |

### Preparation Examples 7 to 21 of Second Solid Freeform Fabrication Liquid Material

### Preparation of Second Solid Freeform Fabrication Liquid Material 7 to 21

Second solid freeform fabrication liquid materials 7 to 21 were obtained in the same manner as in the Preparation Example 5 of the second solid freeform fabrication liquid material except that the respective compositions were changed as shown in Table 2.

Compositions of the second solid freeform fabrication liquid materials 1 to 21 are shown in Table 2.

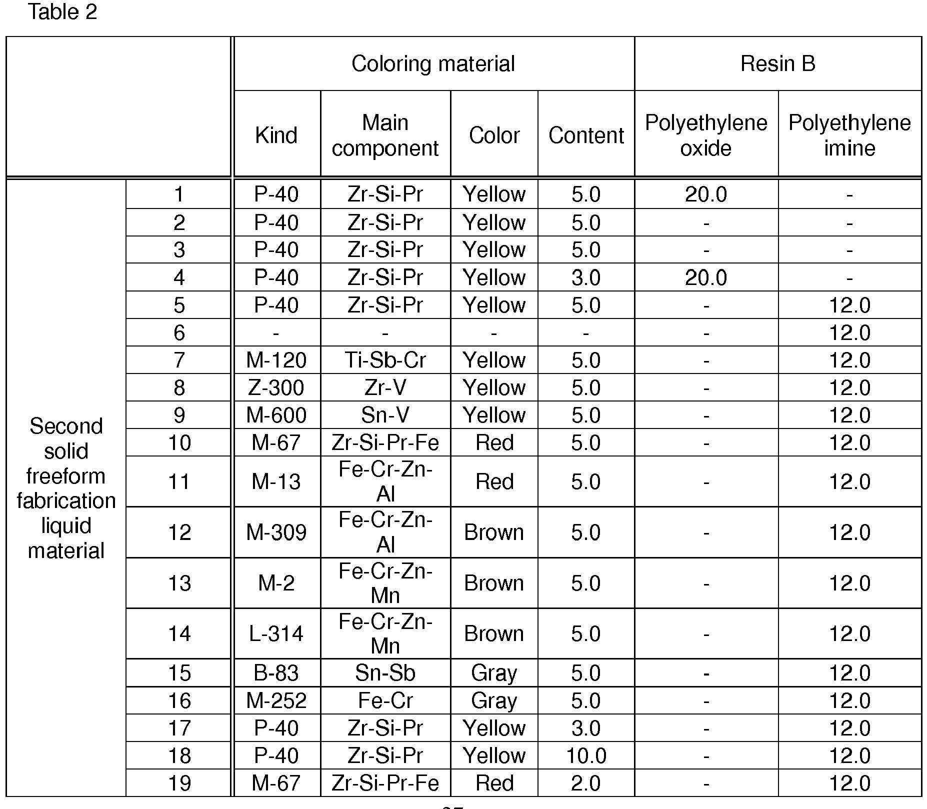

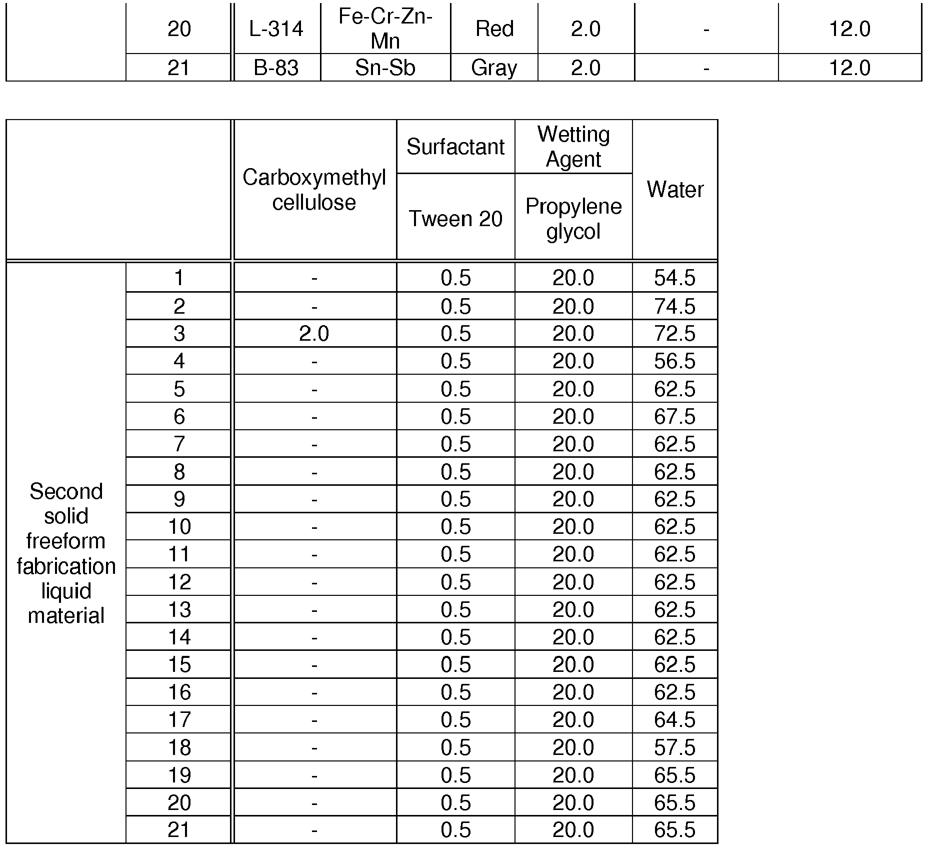

In Table 2, the product name and the manufacturing company of the ingredients are as follows:
- Polyethylene oxide (PEO, ALKOX L-8, manufactured by Meisei Chemical Works, Ltd.)
- Carboxymethyl cellulose (CMC DAICEL 1350, manufactured by DAICEL FINECHEM LTD.)
- Polyethylene imine (PEI) (SP-200, manufactured by Nippon Shokubai Co., Ltd.)
- P-40, M-120, Z-300, and M-600 (yellow pigment, manufactured by NITTO GANRYO KOGYO CO., LTD.)
- M-67 and M-13 (red pigment, manufactured by NITTO GANRYO KOGYO CO., LTD.)
- M-309, M-2, and L314 (brown pigment, manufactured by NITTO GANRYO KOGYO CO., LTD.)
- B-83 and M-252 (gray pigment, manufactured by NITTO GANRYO KOGYO CO., LTD.)

### Example 1

The thus-obtained first solid freeform fabrication liquid material (slurry material) and the second solid freeform fabrication liquid material 1 were paired to form a solid freeform fabrication material set 1. Using the solid freeform fabrication set 1, a solid freeform fabrication object (additive manufacturing object) 1 was manufactured in the following operations 1 to 3 based on a shape print pattern having a size of 70 mm long × 12 mm wide. The compositions were shown in Table 3.
1. Using a device for manufacturing a solid freeform object as illustrated in FIG. 1, the slurry material 1 was conveyed from the storage tank on the supply side to the storage tank on the fabrication side and a thin layer of the slurry material 1 having an average thickness of 100 µm was formed on the substrate mentioned above.
2. Next, the second solid freeform fabrication liquid material 1 was applied (discharged) from the nozzle of an inkjet printer (SG3100KE, manufactured by Ricoh Company Ltd.) to the surface of the thin layer of the slurry material 1 to cure the slurry material 1. The mass ratio of the ink to be discharged to the slurry was set to 1 to 9.
3. Next, the operations 1 and 2 mentioned above were repeated to sequentially laminate the thin layers of the cured slurry material 1 until the total average thickness was a predetermined value, i.e., 3 mm, in order to fabricate a cured object. The thus-obtained cured object was left undone at room temperature to evaporate the solvent. Subsequently, the non-fabrication part was removed with a spoon, a brush, etc. to manufacture a solid freeform fabrication object 1.
   The solid freeform fabrication object obtained in the operation 3 was subject to sintering treatment to manufacture a sintered compact of the solid freeform fabrication object after sintering as in the operation 4 below.
4. The solid freeform fabrication object was subject to sintering treatment at 1,500 degrees C in the atmosphere environment in a sintering furnace. The sintered compact of the solid freeform fabrication object was a completely integrated structure and not broken at all when slammed down on a hard floor.

### Examples 2 to 20 and Comparative Examples 1 to 7

Solid freeform fabrication objects (additive manufacturing objects) 2 to 27 were obtained in the same manner as in Example 1 except that the first solid freeform fabrication liquid material 1 and the second solid freeform fabrication liquid material 1 were changed to the combinations shown in Table 3. The compositions were shown in Table 3.

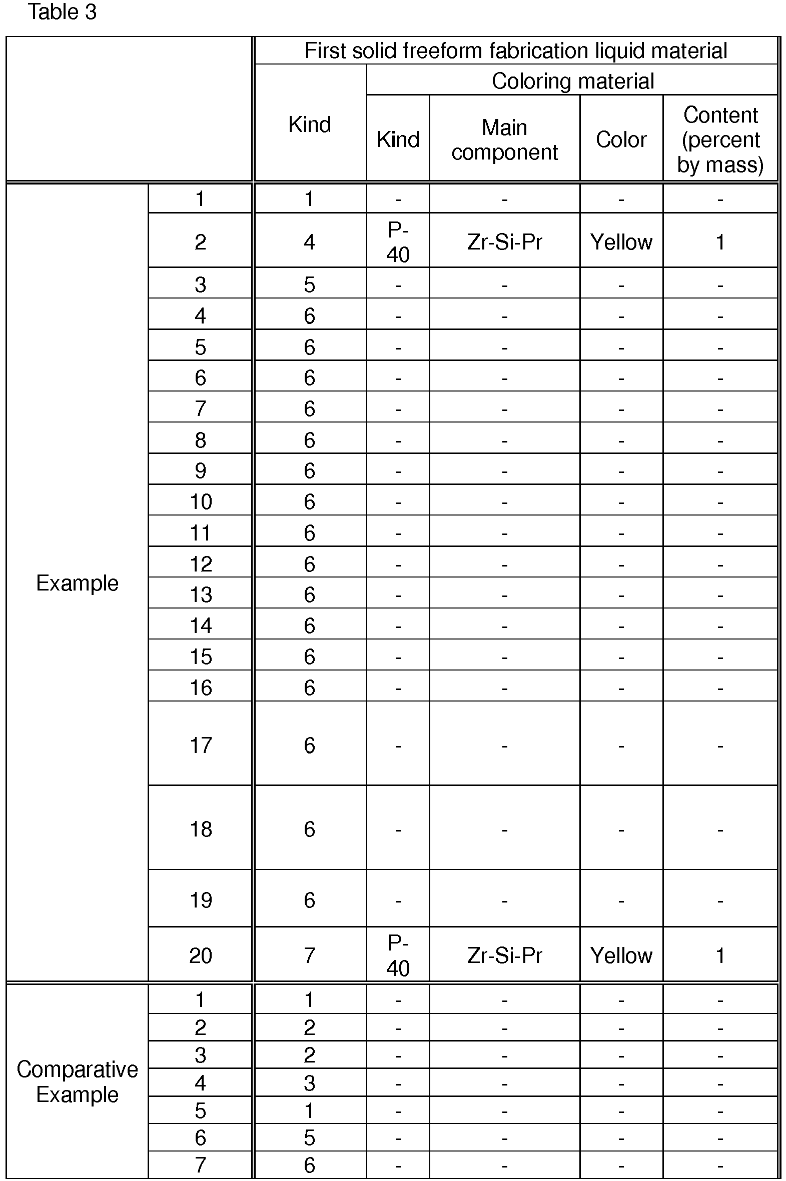

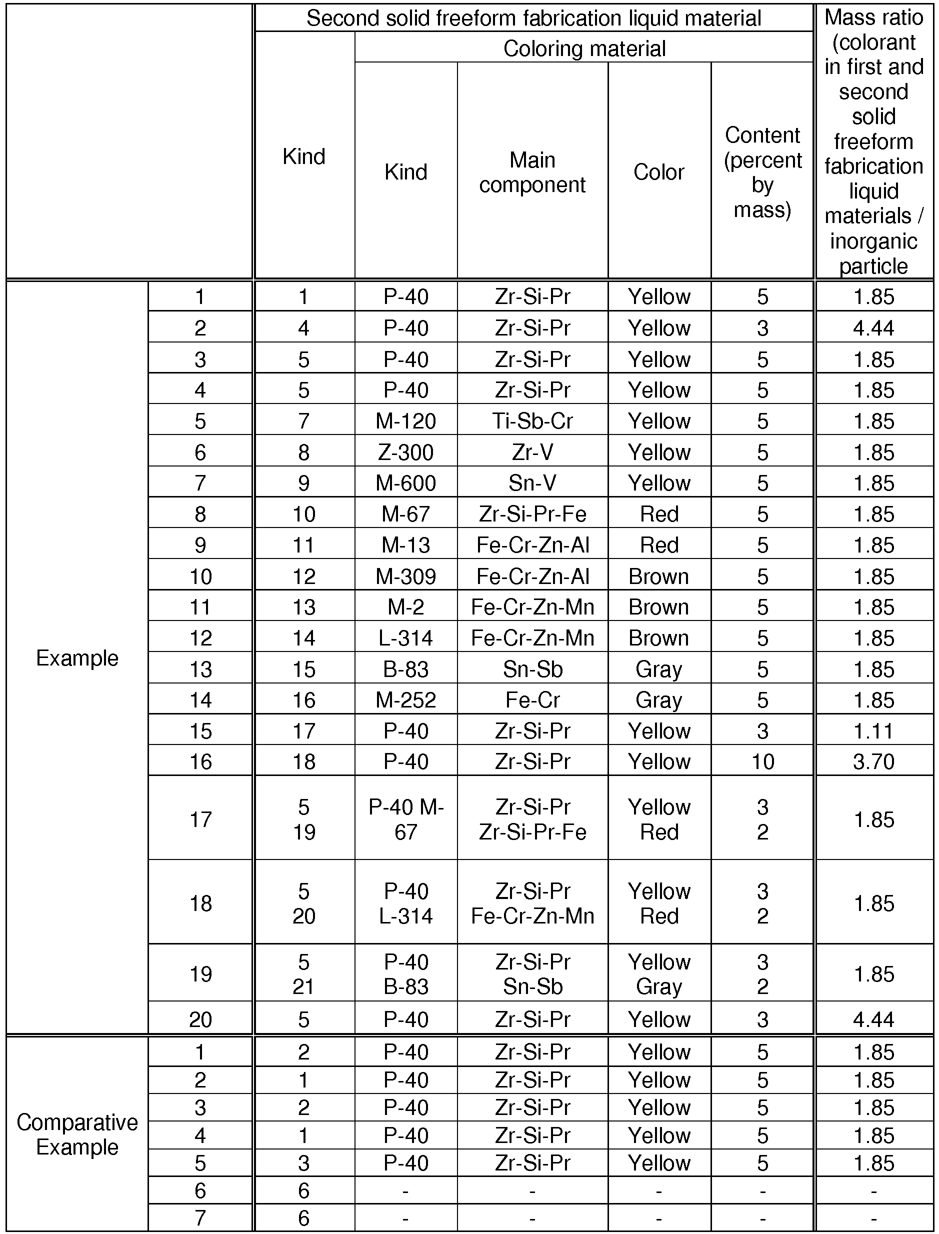

With regard to the sintered compact of the solid freeform fabrication object after sintering of Examples 1 to 20 and Comparative Examples 1 to 7, bending strength after sintering, color space, color uniformity, and discharging stability were evaluated. The results are shown in Table 4.

### Bending Strength After Sintering

Using AUTOGRAPH-AGS-J, manufactured by Shimadzu Corporation, bending strength after sintering was measured according to ISO-6871. Enabling level of bending strength after sintering was 210 MPa or higher.

### Color Space

All the optical values of L*, a*, and b* obtained in Examples were measured using a spectrophotometer (CM-3700A, manufactured by KONICA MINOLTA, INC.) with a measuring diameter of 8 mm according to the standard of DIN 5033 or DIN 6174. The measuring value was defined as the average of the measuring results of the center and right and left ends for both sides (6 sites in total) to evaluate color space according to the evaluation criteria. The fabricated object preferably has optical values close to those of natural teeth.

### Evaluation Criteria

L* is preferably +95 or less, more preferably +85 to +50, and particularly preferably from +75 to +55.

a* is preferably +10 to -10 for ceramic particle.

Metal is preferably +30 to +10.

b* is preferably +5 or greater and more preferably from +15 to +35.

### Color Uniformity

Using a cutter ("PRO waidoserarohtasu GT" GT-150, manufactured by ISHII TOOLS MFG. CO., LTD.), the thus-obtained solid freeform fabrication object was cut in the direction perpendicular to the lamination surface and the colorant in the lamination cross section) was observed with an optical microscope (VHX-5000, manufactured by KEYENCE CORPORATION). The number of particles and the maximum diameter (long side of the shape) of the agglomerated colorant are shown when observed per 1 square cm.

### Discharging Stability

Using the thus-obtained solid freeform fabrication material set, six solid freeform fabrication objects (additive manufacturing objects) were manufactured per hour and fabrication was conducted eight hours per day. Discharging was checked one day, seven days, and thirty days after the fabrication to evaluate discharging stability according to the following evaluation criteria.

### Evaluation Criteria

G (Good): No non-discharging nozzle
M (Marginal): There are non-discharging nozzles but cleaning is effective to dissolve non-discharging.
M (Marginal): There are non-discharging nozzles and cleaning is not effective to dissolve non-discharging.

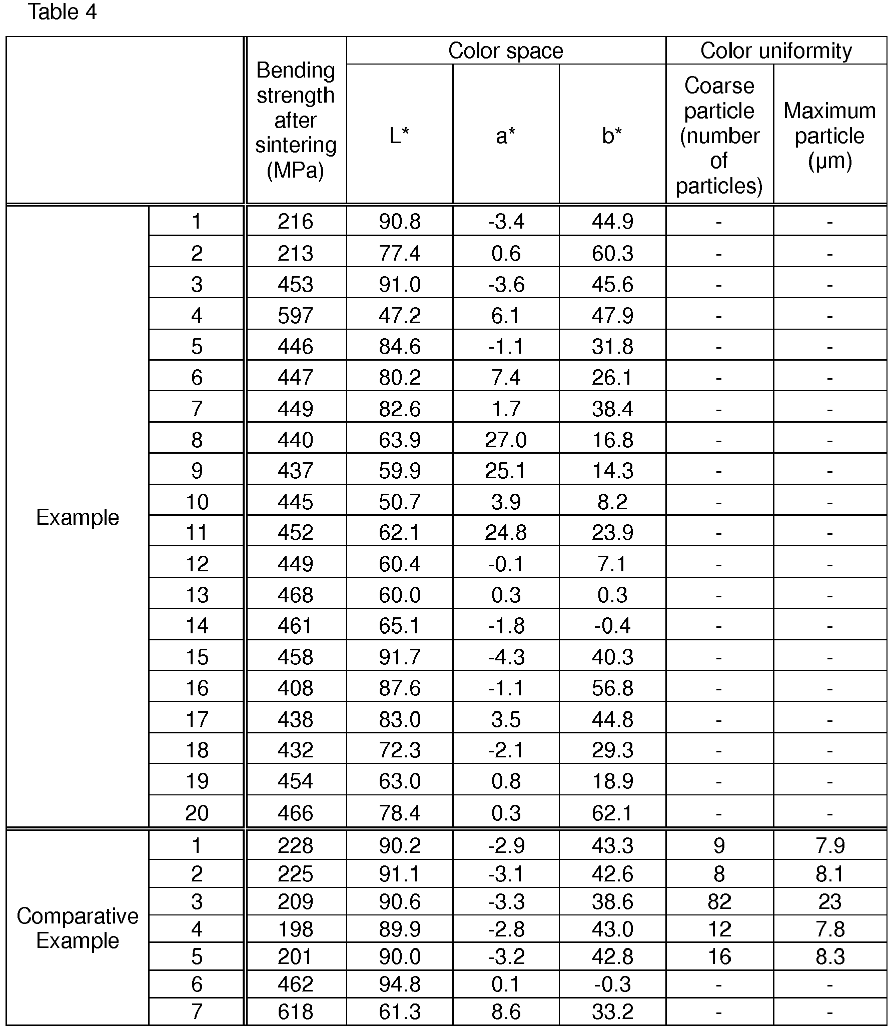

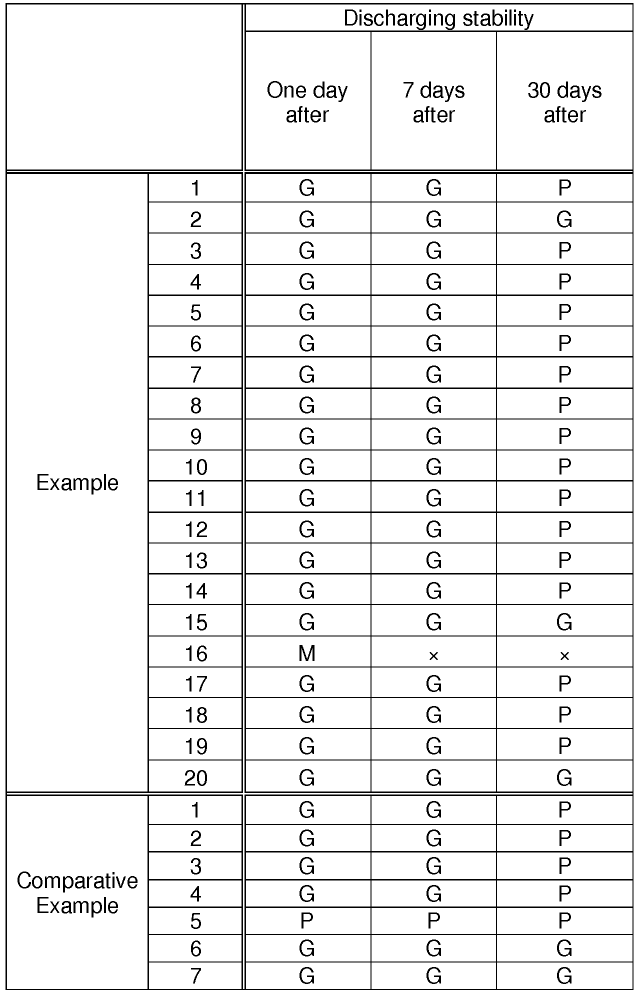

More colored fabrication object was obtained with discharging stability sustained for a longer period of time in Example 2 than in Example 1.

Non-white zirconia solid freeform fabrication object was obtained in Example 3 in comparison with Comparative Example 6.

Colored titanium solid freeform fabrication object was obtained in Example 4 in comparison with Comparative Example 7.

In Comparative Example 5, although the resin was added to the upper limit of solution to prepare the second solid freeform fabrication liquid material, color uniformity was not improved but nozzle clogging occurred to the contrary.

The solid freeform fabrication objects in Examples 1 to 20 had non-white colorant disposed at desired sites in three dimensional manner and color gradation was added.

In Examples 1 to 20 and Comparative Examples 6 and 7, no agglomeration of the colorant was observed.

In Comparative Examples 1 to 3, no color uniformity was obtained unless at least one of the first solid freeform fabrication liquid material and the second solid freeform fabrication liquid material included resins soluble in solvent.

Aspects of the present disclosure are, for example, as follows.
1. A solid freeform fabrication material set including
   a first solid freeform fabrication liquid material including a solvent, a resin A soluble in the solvent, and an inorganic particle and a second solid freeform fabrication liquid material including a resin B soluble in water and a colorant.
2. The solid freeform fabrication material set according to 1 mentioned above, wherein the first solid freeform fabrication liquid material includes a colorant.
3. The solid freeform fabrication material set according to 2 mentioned above, wherein the mass ratio of the second solid freeform fabrication liquid material is higher than the mass ratio of the colorant to the first solid freeform fabrication liquid material.
4. The solid freeform fabrication material set according to any one of 1 to 3, wherein the resin B is reactive with the resin A.
5. The solid freeform fabrication material set according to any one of 1 to 3, wherein the resin B is acid-base reactive with the resin A.
6. The solid freeform fabrication material set according to any one of 1 to 5, wherein the resin B is a polyacrylic acid.
7. The solid freeform fabrication material set according to any one of 1 to 6, wherein the resin B is a polyethylene imine.
8. The solid freeform fabrication material set according to any one of 1 to 7, wherein the inorganic particle includes at least one of a ceramic particle and a metal particle.
9. The solid freeform fabrication material set according to any one of 1 to 8, wherein the inorganic particle is biocompatible.
10. The solid freeform fabrication material set according to 8 or 9 mentioned above, wherein the volume average particle diameter of the ceramic particle in the first solid freeform fabrication liquid material is less than 5 µm.
11. The solid freeform fabrication material set according to any one of 8 to 10 mentioned above, wherein the volume average particle diameter of the inorganic particle in the first solid freeform fabrication liquid material is less than 50 µm.
12. The solid freeform fabrication material set according to any one of 1 to 11 mentioned above, wherein the content ratio of the inorganic material is 20 to 70 parts by mass to 100 parts by mass of the first solid freeform fabrication liquid material.
13. The solid freeform fabrication material set according to any one of 1 to 12 mentioned above, wherein the solvent includes an organic solvent.
14. The solid freeform fabrication material set according to 13 mentioned above, wherein organic solvent includes ethanol.
15. A method of manufacturing a solid freeform fabrication object, including forming a first solid freeform fabrication liquid material layer using the first solid freeform fabrication liquid material in the solid freeform fabrication material set of any one of 1 to 14 mentioned above, applying the second solid freeform fabrication liquid material in the solid freeform fabrication material set of any one of 1 to 14 mentioned above to a predetermined area of the first solid freeform fabrication liquid material layer, and
   repeating the forming and applying multiple times.
16. The method according to 15 mentioned above, including evaporating the solvent after forming the layer.
17. The method according to 15 or 16 mentioned above, including removing uncured portions of the first solid freeform fabrication liquid material layer by liquid immersion after fabrication.
18. The method according to any one of 15 to 17 mentioned above, manufacturing dental prosthesis.
19. A device for manufacturing a solid freeform fabrication object, including a layer forming device to form a first solid freeform fabrication liquid material layer using the first solid freeform fabrication liquid material in the solid freeform fabrication material set of any one of 1 to 14 mentioned above and a liquid application device to apply the second solid freeform fabrication liquid material in the solid freeform fabrication material set of any one of 1 to 14 mentioned above to a predetermined area of the first solid freeform fabrication liquid material layer.
20. A dental prosthesis manufactured according to the device for manufacturing a solid freeform fabrication object of 19 mentioned above.

According to the present disclosure, a solid freeform fabrication material set is provided with which a complex solid freeform fabrication object having non-white colorants disposed therein in three-dimensional manner can be simply and stably manufactured using a material having a high melting point and high hardness.

Having now fully described embodiments of the present invention, it will be apparent to one of ordinary skill in the art that many changes and modifications can be made thereto without departing from the spirit and scope of embodiments of the invention as set forth herein.

## Claims

1. A solid freeform fabrication material set comprising:
a first solid freeform fabrication liquid material including a solvent, a first resin soluble in the solvent such that 10% by mass or greater dissolves in the solvent at 25 °C, and an inorganic particulate material; and
a second solid freeform fabrication liquid material including a second resin soluble in water such that 10% by mass or greater dissolves in water at 25 °C, and a colorant, wherein the second resin is reactive with the first resin.

2. The solid freeform fabrication material set according to claim 1, wherein the colorant is an oxide of a rare earth metal or a transition metal.

3. The solid freeform fabrication material set according to claim 1, or 2, wherein the first solid freeform fabrication liquid material further includes a colorant.

4. The solid freeform fabrication material set according to claim 3, wherein a mass ratio of the colorant in the second solid freeform fabrication liquid material to that material is higher than a mass ratio of the colorant in the first solid freeform fabrication liquid material to that material.

5. The solid freeform fabrication material set according to any one of claims 1 to 4, wherein the proportion of colorant is 1.0 to 5.0 percent by mass of the entire amount of the second solid freeform fabrication liquid material.

6. The solid freeform fabrication material set according to any one of claims 1 to 5, wherein the inorganic particulate material includes at least one of a ceramic particulate material and a metal particulate material.

7. The solid freeform fabrication material set according to any one of claims 1 to 6, wherein the inorganic particulate material is biocompatible.

8. The solid freeform fabrication material set according to claim 6 or 7, wherein a volume average particle diameter of the ceramic particulate material in the first solid freeform fabrication liquid material is less than 5 µm and a volume average particle diameter of the metal particulate material in the first solid freeform fabrication liquid material is less than 50 µm.

9. The solid freeform fabrication material set according to any one of claims 1 to 8, wherein a content ratio of the inorganic particulate material is 20 to 70 parts by mass to 100 parts by mass of the first solid freeform fabrication liquid material.

10. A method of manufacturing a solid freeform fabrication object, comprising:
forming a first solid freeform fabrication liquid material layer with a first solid freeform fabrication liquid material including a solvent, a first resin soluble in the solvent such that 10% by mass or greater dissolves in the solvent at 25 °C, and an inorganic particulate material;
applying a second solid freeform fabrication liquid material including a second resin soluble in water such that 10% by mass or greater dissolves in water at 25 °C, and a colorant, to an area of the first solid freeform fabrication liquid material layer, wherein the second resin is reactive with the first resin; and
repeating the forming and the applying multiple times.

11. The method according to claim 10, further comprising:
evaporating the solvent after the forming.

12. The method according to claim 10 or 11, further comprising
removing an uncured portion of the first solid freeform fabrication liquid material by liquid immersion after the repeating.

13. The method according to any one of claims 10 to 12, further comprising sintering the solid freeform fabrication object after the repeating.

14. The method according to any one of claims 10 to 13, wherein the solid freeform fabrication object is a dental prosthesis.

## Patentansprüche

1. Fest-Freiformfertigungsmaterialsatz umfassend:
ein erstes Fest-Freiformfertigungs-Flüssigmaterial, das ein Lösungsmittel, ein erstes Harz, das in dem Lösungsmittel derart löslich ist, dass 10 Masse-% oder mehr sich in dem Lösungsmittel bei 25 °C lösen, und ein anorganisches teilchenförmiges Material umfasst; und
ein zweites Fest-Freiformfertigungs-Flüssigmaterial, das ein zweites Harz, das in Wasser derart löslich ist, dass 10 Masse-% oder mehr sich in Wasser bei 25 °C lösen, und ein Farbmittel umfasst,
wobei das zweite Harz mit dem ersten Harz reaktionsfähig ist.

2. Fest-Freiformfertigungsmaterialsatz nach Anspruch 1, wobei das Farbmittel ein Oxid eines Seltenerdmetalls oder eines Übergangsmetalls ist.

3. Fest-Freiformfertigungsmaterialsatz nach Anspruch 1 oder 2, wobei das erste Fest-Freiformfertigungs-Flüssigmaterial ferner ein Farbmittel umfasst.

4. Fest-Freiformfertigungsmaterialsatz nach Anspruch 3, wobei ein Masseverhältnis des Farbmittels in dem zweiten Fest-Freiformfertigung-Flüssigmaterial zu diesem Material höher als ein Masseverhältnis des Farbmittels in dem ersten Fest-Freiformfertigungs-Flüssigmaterial zu diesem Material ist.

5. Fest-Freiformfertigungsmaterialsatz nach einem der Ansprüche 1 bis 4, wobei der Anteil des Farbmittels 1,0 bis 5,0 Masseprozent der Gesamtmenge des zweiten Fest-Freiformfertigungs-Flüssigmaterials beträgt.

6. Fest-Freiformfertigungsmaterialsatz nach einem der Ansprüche 1 bis 5, wobei das anorganische teilchenförmige Material mindestens eines von einem teilchenförmigen Keramikmaterial und einem teilchenförmigen Metallmaterial umfasst.

7. Fest-Freiformfertigungsmaterialsatz nach einem der Ansprüche 1 bis 6, wobei das anorganische teilchenförmige Material biokompatibel ist.

8. Fest-Freiformfertigungsmaterialsatz nach Anspruch 6 oder 7, wobei ein volumendurchschnittlicher Teilchendurchmesser des teilchenförmigen Keramikmaterials in dem ersten Fest-Freiformfertigungs-Flüssigmaterial weniger als 5 µm beträgt und ein volumendurchschnittlicher Teilchendurchmesser des teilchenförmigen Metallmaterials in dem ersten Fest-Freiformfertigungs-Flüssigmaterial weniger als 50 µm beträgt.

9. Fest-Freiformfertigungsmaterialsatz nach einem der Ansprüche 1 bis 8, wobei ein Gehaltsverhältnis des anorganischen teilchenförmigen Materials 20 bis 70 Masseteile zu 100 Masseteilen des ersten Fest-Freiformfertigungs-Flüssigmaterials beträgt.

10. Verfahren zum Fertigen eines Fest-Freiform-Fertigungsobjekts, umfassend:
Bilden einer ersten Fest-Freiformfertigungs-Flüssigmaterialschicht mit einem ersten Fest-Freiformfertigungs-Flüssigmaterial, das ein Lösungsmittel, ein erstes Harz, das in dem Lösungsmittel derart löslich ist, dass 10 Masse-% oder mehr sich in dem Lösungsmittel bei 25 °C lösen, und ein anorganisches teilchenförmiges Material umfasst;
Aufbringen eines zweiten Fest-Freiformfertigungs-Flüssigmaterials, das ein zweites Harz, das in Wasser derart löslich ist, dass 10 Masse-% oder mehr sich in Wasser bei 25 °C lösen, und ein Farbmittel umfasst, auf einen Bereich der ersten Fest-Freiformfertigungs-Flüssigmaterialschicht, wobei das zweite Harz mit dem ersten Harz reaktionsfähig ist; und
Wiederholen des Bildens und Aufbringens mehrere Male.

11. Verfahren nach Anspruch 10, ferner Folgendes umfassend:
Verdampfen des Lösungsmittels nach dem Bilden.

12. Verfahren nach Anspruch 10 oder 11, ferner Folgendes umfassend
Entfernen eines unausgehärteten Teils des ersten Fest-Freiformfertigungs-Flüssigmaterials durch in Flüssigkeit Tauchen nach dem Wiederholen.

13. Verfahren nach einem der Ansprüche 10 bis 12, ferner das Sintern des Fest-Freiformfertigungsobjekts nach dem Wiederholen umfassend.

14. Verfahren nach einem der Ansprüche 10 bis 13, wobei das Fest-Freiformfertigungsobjekt eine Zahnprothese ist.

## Revendications

1. Ensemble de matériaux de fabrication de forme libre solide comprenant:
un premier matériau liquide de fabrication de forme libre solide comprenant un solvant, une première résine soluble dans le solvant de sorte que 10 % en masse ou plus se dissout dans le solvant à 25°C, et un matériau sous forme de particules inorganique; et
un second matériau liquide de fabrication de forme libre solide comprenant une seconde résine soluble dans l'eau de sorte que 10 % en masse ou plus se dissout dans l'eau à 25°C, et un colorant,
la seconde résine étant réactive avec la première résine.

2. Ensemble de matériaux de fabrication de forme libre solide selon la revendication 1, dans lequel le colorant est un oxyde d'un métal de terres rares ou d'un métal de transition.

3. Ensemble de matériaux de fabrication de forme libre solide selon la revendication 1 ou 2, le premier matériau liquide de fabrication de forme libre solide comprenant en outre un colorant.

4. Ensemble de matériaux de fabrication de forme libre solide selon la revendication 3, dans lequel un rapport en masse du colorant dans le second matériau liquide de fabrication de forme libre solide à ce matériau est supérieur à un rapport en masse du colorant dans le premier matériau liquide de fabrication de forme libre solide à ce matériau.

5. Ensemble de matériaux de fabrication de forme libre solide selon l'une quelconque des revendications 1 à 4, la proportion du colorant étant de 1,0 à 5,0 pour cent en masse de la quantité entière du second matériau liquide de fabrication de forme libre solide.

6. Ensemble de matériaux de fabrication de forme libre solide selon l'une quelconque des revendications 1 à 5, le matériau sous forme de particules inorganique comprenant au moins l'un d'un matériau sous forme de particules de céramique et d'un matériau sous forme de particules métalliques.

7. Ensemble de matériaux de fabrication de forme libre solide selon l'une quelconque des revendications 1 à 6, le matériau sous forme de particules inorganique étant biocompatible.

8. Ensemble de matériaux de fabrication de forme libre solide selon la revendication 6 ou 7, dans lequel un diamètre de particule moyen en volume du matériau sous forme de particules de céramique dans le premier matériau liquide de fabrication de forme libre solide est inférieur à 5 µm et un diamètre de particule moyen en volume du matériau sous forme de particules métalliques dans le premier matériau liquide de fabrication de forme libre solide est inférieur à 50 µm.

9. Ensemble de matériaux de fabrication de forme libre solide selon l'une quelconque des revendications 1 à 8, dans lequel un rapport des teneurs du matériau sous forme de particules inorganique est de 20 à 70 parties en masse à 100 parties en masse du premier matériau liquide de fabrication de forme libre solide.

10. Procédé de fabrication d'un objet de fabrication de forme libre solide, comprenant:
la formation d'une première couche de matériau liquide de fabrication de forme libre solide avec un premier matériau liquide de fabrication de forme libre solide comprenant un solvant, une première résine soluble dans le solvant de sorte que 10 % en masse ou plus se dissout dans le solvant à 25°C, et un matériau sous forme de particules inorganique;
l'application d'un second matériau liquide de fabrication de forme libre solide comprenant une seconde résine soluble dans l'eau de sorte que 10 % en masse ou plus se dissout dans l'eau à 25°C, et un colorant, à une surface de la première couche de matériau liquide de fabrication de forme libre solide, dans laquelle la seconde résine est réactive avec la première résine; et
la répétition de multiples fois de la mise en forme et de l'application.

11. Procédé selon la revendication 10, comprenant en outre:
l'évaporation du solvant après la mise en forme.

12. Procédé selon la revendication 10 ou 11, comprenant en outre
l'élimination d'une partie non durcie du premier matériau liquide de fabrication de forme libre solide par immersion dans un liquide après la répétition.

13. Procédé selon l'une quelconque des revendications 10 à 12, comprenant en outre le frittage de l'objet de fabrication de forme libre solide après la répétition.

14. Procédé selon l'une quelconque des revendications 10 à 13, dans lequel l'objet de fabrication de forme libre solide est une prothèse dentaire.
